(19) Europäisches Patentamt
European Patent Office
Office européen des brevets

(11) **EP 4 579 674 A1**

(12) **EUROPEAN PATENT APPLICATION**

(43) Date of publication:
**02.07.2025 Bulletin 2025/27**

(21) Application number: **23220563.3**

(22) Date of filing: **28.12.2023**

(51) International Patent Classification (IPC):
***G16H 20/30*** *(2018.01)*

(52) Cooperative Patent Classification (CPC):
**G16H 20/30;** G16H 20/70; G16H 40/67;
G16H 50/20; G16H 50/70; G16H 80/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Blended Clinic AI GmbH
90411 Nürnberg (DE)**

(72) Inventor: **CRÜTS, Björn
6221 Maastricht (NL)**

(74) Representative: **Prüfer & Partner mbB
Patentanwälte · Rechtsanwälte
Sohnckestraße 12
81479 München (DE)**

(54) **METHOD AND SYSTEM FOR REMOTELY MONITORING PATIENTS**

(57) A method for remotely monitoring a patient, comprises:
providing an online portal to a medical professional enabling him to
- assign an exercise program to the patient, wherein the exercise program consists of a number of exercises to be completed by the patient each time in regular time intervals and
- prompting the patient to send feedback data via a communication network,

wherein patient's compliance to the exercise program is predicted by a machine learning (ML) model based on a gradient boosting algorithm,
wherein the machine learning model analyses the feedback data of a patient and predicts the compliance when performing the exercise program the next time after expiry of a number of time intervals to follow.

**Fig. 7**

**Description**

The present invention refers to a method for remotely monitoring a patient, a corresponding machine learning (ML) model, a corresponding method for improving a level of patient compliance and the corresponding use of a method for remotely monitoring a patient in therapeutic treatment or for improving a level of patient compliance.

In recent years, there has been a significant increase in the worldwide occurrence of strokes, imposing a substantial strain on healthcare systems globally. The consequences of strokes reach beyond their immediate effects, profoundly impacting the daily lives of survivors and frequently resulting in a significant decline in their overall well-being.

Medical interventions can lead to considerable improvements in the quality of life experienced by individuals who have survived strokes. However, in view of the number of patients in need of medical interventions, a severe financial burden rests on the healthcare system in a country and on healthcare centers able to provide the required treatment, leading e.g. to long patient waiting lists. Here, online therapy comes into play. Though being more cost-effective than conventional treatment, it allows for scalable, personalized strategies, in addition to the noteworthy benefit of eliminating the need for patients to commute as they can access therapy and support from the comfort of their homes. However, as in online therapy the patient is remote from the therapist, there remains the problem of supervising a patient's adherence to the therapy program, having in mind that adherence to the therapy regimens is crucial for successful recovery and improved patient outcomes.

Accordingly, it is an object of the present invention to provide a method and device assisting a healthcare professional in a remote management of patients, in particular stroke patients.

The object is achieved by a method according to claim 1 or claim 11, by a corresponding use of such a method in accordance with claim 14 or 15 and by a machine learning (ML) model according to claim 9.

An inventive method for remotely monitoring a patient comprises:

providing an online portal to a medical professional enabling him to

- assign an exercise program to the patient, wherein the exercise program consists of a number of exercises to be completed by the patient each time in regular time intervals and
- prompting the patient to send feedback data via a communication network,

wherein patient's compliance to the exercise program is predicted by a machine learning (ML) model based on a gradient boosting algorithm,
wherein the machine learning model analyses the feedback data of a patient and predicts the compliance when performing the exercise program the next time after expiry of a number of time intervals to follow.

Here, the term "remote" means that the patient and the medical professional, e.g. a therapist, are not meeting each other in person. The term "monitoring" means that the medical status of the patient, in particular the patient's compliance with a therapy program or with parts thereof, is assessed.

An exercise program may be considered as part of a therapy program, which is prescribed for the patient and comprises a number of exercises to be executed each time in regular time intervals. A time interval may in particular be one day, a plurality of days, a week or also a number of hours. In particular, the term "in regular time intervals" may also comprise a number of exercises to be completed by a patient a number of times a day, but not during the night.

The feedback data sent by the patient via a communication network, which is a telecommunications network, in particular the Internet, comprise in particular information on the patient's performance after having done the number of exercises. Feedback data can be sent e.g. each time after the completion of an exercise. In particular, the communication with the patient can be implemented such that the patient is connected by means of a client device such as a smartphone or tablet to a server, on which the machine learning model is implemented and to which the medical professional is also connected by means of a client device.

The term "compliance" is usually understood to describe the accurateness with which a patient sticks to a prescribed therapy. How it is defined in detail, does not affect the inventive approach, though the definition may affect therapy success. The compliance may e.g. be defined based on

- the percentage of days on which the exercises are skipped

- the percentage of exercises executed per day

- the average time spent per exercise

- the percentage of exercises for which no feedback is given after their completion or

- the average degree of completion of an exercise based on all exercises prescribed e.g. for a day.

The predicted compliance may e.g. be a binary information indicating "will comply" and "will not comply" depending e.g. on whether a patient will successfully complete over 50% of the prescribed exercises or less than 50 %.

The term "the next time after expiry of a number of time intervals to follow" may e.g. refer to the next day, the day after the next day, etc. In this application, the term "a number of" generally means "one or more". Preferably, the model predicts the compliance at the time the patient is to perform the exercise program the next time after expiry of the next time interval.

In an aspect of the invention, the compliance is predicted by determining the likelihood of a patient completing more than 50%, preferably more than 70%, of the number of exercises when performing the exercise program the next time after expiry of a number of time intervals to follow, preferably after expiry of the next time interval.

As it was the case for the term "compliance", there is more than one definition for the term "completion". Again, how the term is defined in detail, does not affect the inventive approach, though the definition may affect therapy success.

When stating that a patient has completed a certain percentage of the number of exercises e.g. on a day, this may refer to the percentage of exercises the patient has executed to the full extent. In order to assess whether a patient has executed an exercise to the full extent, it is e.g. possible to specify a time interval, within which the patient shall execute the exercise. If the time the patient has needed does not lie within the specified time interval (meaning the patient needed more time or less time) than by definition the respective exercise could be considered as having not been executed to the full extent. Alternatively, the statement may refer to the average percentage of execution of the exercises calculated from the percentages (degrees) by which each of the exercises on that day has been executed. This applies to situations in which e.g. an exercise has not been executed to the full extent.

For example, with the exercise program being performed day by day, compliance could be defined as follows: A patient is compliant, if X % of exercises are completed on that day. Accordingly, one could define a non-compliance to exist, when the patient is predicted to do less than X % of the exercises on the day to follow. Here, the percentages 50%, 70% and 90% can be used to define three categories:

50% - 70%: medium compliance;

70% - 90%: good compliance;

larger than 90% excellent compliance.

According to a further aspect of the invention, a motivation message is sent to a patient for the case that a low compliance is predicted.

Any motivation message, meaning a message that is encouraging the patient to perform the exercises, can be sent to the patient's client device. Either the message can be sent by the medical professional that has been informed on the patient's predicted compliance or the message can be sent automatically by the server, when the machine learning model has predicted a low compliance in the future. Preferably, the message is sent such that the patient is able to read it next time when starting to perform the number of exercises, wherein it is assumed that the patient's client device guides the patient when performing the exercises so that it is actually used by the patient at that time.

What is considered to be a low predicted compliance may depend on the context, e.g. the disability level of the client. In many cases, a low predicted compliance can be considered to be a likelihood below 50 % that a patient will complete more than 50 % of the number of exercises. As above, this may refer to the percentage of exercises the patient has executed to the full extent or to the average percentage calculated from the percentages (degrees) by which each of the exercises are executed.

According to a further aspect of the invention, the feedback data include one or more of the following data:

- a patient identifier and optionally a course identifier,
- on which day of the sequential number of days of the course the feedback data are sent,
- the date,
- patient's subjective evaluation whether the patient complied on that date or not,
- the number of exercises assigned to the patient on that date,
- the painfulness of at least one exercise,
- the difficulty the patient had when carrying out at least one exercise,
- the time needed for at least one of the individual exercises making up the training program,
- information for at least one of the exercises whether it has been completed or not,
- the number of exercises completed per day,
- an exercise identifier,

- information whether an exercise targets an upper or lower body part,
- the day of the week the feedback data are sent,
- the therapy center to which the patient belongs to,
- the actual number of exercises executed by the patient each time,
- which of the number of exercises are skipped,
- the average time per exercise,
- the patient's assessment of the intensity of one or more exercises,
- patient's demographics like age, gender, height and weight,
- optionally physiological parameters such as blood pressure and/or heart rate.

Here, a course can be considered as a number of exercises to be completed by the patient on a regular basis over an extended number of time intervals such as a number of exercises to be performed by the patient on a daily basis for 28 days.

A particular information to be included in the feedback data could be the day of the course on which the feedback data are sent, which is the day of the sequential number of days of the course the feedback data are sent.

A date is considered as a particular day in a particular year.

Though patient's evaluation whether the patient complied on that date or not can be highly subjective, it is suitable for evaluating the patient's motivation. The same applies to other subjective assessments made by the client such as statements on the painfulness or difficulty of an exercise. Also the assessment, whether an exercise has been successfully completed or not, can be influenced by subjective bias.

Nevertheless, it is possible to define an objective criterion for the decision, whether an exercise has been successfully completed or not. Such an objective criterion is based on the time a patient has needed for performing an individual exercise. For example, a default time period between 20 s and e.g. 310 s, sometimes between 50 s and 250 s, can be defined for each individual exercise and a patient can be considered to have successfully completed an exercise, when the time the patient has actually needed for performing the exercise lies within the default time period. In practice, the patient could be prompted to push a button (or do something equivalently) immediately before starting an exercise and immediately after having finished an exercise.

In general, the feedback data should contain information that can change each time the patient performs the number of exercises. Other information that does not change can possibly be derived from a server-based database containing information about the patient such as the course identifiers of the courses assigned to the patient, the date on which the feedback data from the patient are received, the number of exercises assigned to the patient on that date, information on which day of the sequential number of days of the course the feedback data are sent, the day of the week, patient's demographics like age, gender, height and weight, etc.

For example, data set sent back by a patient after having performed the number of exercises assigned to him/her for a particular day may consist of the following information:

- a patient identifier,
- the date,
- a statement, whether the patient had discomfort when performing at least one of the exercises, e.g. pain, excessive exhaustion, a feeling of dizziness, etc.,
- the time the patient needed for performing each of the exercises, wherein the time for a skipped exercise can be considered to be zero.

In addition, the parameters listed above could be complemented by the following:

- the patient's assessment of the intensity of the exercises, e.g. by assigning an integer between 1 and 5 (integers 1 and 5 included) to each exercise, where 1 means very low intensity and 5 means very high intensity,
- the weekday (day of the week),
- an identifier of the therapy center and/or therapist that have assigned the course to the patient.

According to a further aspect of the invention, the machine learning model has been trained using one or more of the following data:

- time needed for at least one of the individual exercises making up the training program,
- information on whether an exercise has been completed or not,
- the number of exercises completed per day,
- an exercise identifier,
- information whether an exercise targets an upper or lower body part,

- the course that the patient is assigned to,
- on which day of the sequential number of days of the course the feedback data, are sent
- the day of the week the exercises were executed,
- the therapy center to which the patient belongs to,
- a patient identifier or a course identifier,
- the length of the time interval,
- the actual number of exercises executed by the patient each time,
- which of the number of exercises are skipped,
- the time needed for at least one of the individual exercises making up the training program,
- patient's demographics like age, gender, height and weight.

With regard to the time needed for performing an exercise, one could either use a specific time period, which the patient actually needed for performing the exercise. Alternatively, one could just use a binary information, meaning whether or not the time the patient needed lay in a specified time interval for the exercise (e.g. between 20s and 310s).

Whether an exercise has been completed or not could either be decided based on a subjective evaluation by the patient or could be based on the time the patient needed for the exercise, e.g. was it in the specified time range (exercise completed) or outside of the time range (exercise not completed).

Information whether an exercise targets an upper or lower body part could be relevant in particular when the patient has deficiencies (e.g. in an upper or lower body part).

Also the course to which a patient is assigned to, in particular the level of the course, may be a compliance indicator, e.g. because the exercises of "high"-level courses could be of particular difficulty when being performed.

The information on which day of the sequential number of days of the course the feedback data, are sent or the information on the weekday can provide valuable input, as e.g. after a number of days of the course have passed, the patient has already become used to the exercises. Also the weekday can have an influence as the inventors have found that the weekday has an influence on the compliance.

The therapy center to which a patient has been assigned to can have an influence as there may be e.g. regional difference between therapy centers.

Also, the length of the time interval lying between two instances in time at which the number of exercises are performed each time can have an influence as it defines a break, within which the patient is able to recover.

The training data can comprise information that can change each time the patient performs the number of exercises and other information that does not change. Accordingly, the training data will include some of the feedback data and possibly other data from a server-based database. A preferable training data set is the following:

- a patient identifier, optionally a treatment-related identifier of the patient, meaning a course-specific identifier of the patient
- optionally an identifier on the therapy center to which the patient belongs and/or on the therapist
- the day of the course (meaning on which day of the sequential number of days of the course the feedback data were sent)
- a course identifier
- the number of exercises assigned to the patient on a specific instance in time such as a specific day where the number of exercises shall be performed each day
- date-related information like the day of the month, the month and the year
- the compliance status (the target variable)

Here, the compliance status can be a binary variable (compliant or not compliant). It can also correspond to the patient's own subjective assessment, whether the patient complied at a specific instance in time, e.g. on a specific day. Compliance can e.g. be defined based on whether the patient completes more than half of the number of exercises.

Alternatively, the following set of parameters could be used for training the model:

- a patient identifier, optionally a treatment-related identifier of the patient, meaning a course-specific identifier of the patient
- a course identifier
- an identifier on the therapy center to which the patient belongs and/or on the therapist
- the day of the course (meaning on which day of the sequential number of days of the course the feedback data were sent)
- The body part targeted by the number of exercises on a specific day
- the disability level of the patient
- the day of the week (weekday)

- the compliance status (the target variable)

Here, a disability level can be assigned to a patient making use of the modified Rankin Scale (mRS) defined in more detail further below.

According to a further aspect of the invention, at least one of the following parameters could additionally be used for training the model:

- the patient's demographics like age, gender, height and/or weight
- physiological parameters such as blood pressure (systolic and/or diastolic) and/or heart rate,
- at least one activity indicator, in particular the number of steps taken by the patient on the day the patient performs the number of exercises or on the day before,
- at least one vitality indicator.

An example of a vitality indicator would be hand strength and grip strength, respectively, which can be gathered by a specific device. Other vitality parameters could be gathered through questionnaires or monitoring devices.

According to a further aspect of the invention, the machine learning model determines a level of patient compliance based on how much time the patient spent on each exercise and whether the exercise was actually performed or not i.e. skipped.

This means in particular that a time interval is specified, within which the patient is to execute an exercise. If the time the patient has needed does not lie within the specified time interval (meaning the patient needed more time or less time) than the respective exercise by definition could be considered as having been skipped. With such definition, the compliance level for the number of exercises can be considered to be the percentage of exercises that are not skipped.

Alternatively, for example, if an exercise has to be repeated ten times but within the specified time interval the patient is able to repeat it only seven times, a compliance level of 70 % could be assumed and the average of all compliance levels determined in this way could be defined as compliance level for the number of exercises.

A specified time interval can e.g. cover the range between 20 s and 310 s.

According to a further aspect of the invention, the ML model has been trained to determine a level of patient compliance by means of exemplary feedback data.

Here, exemplary feedback data are understood to be feedback data gathered from patients either when training the machine learning model before using it or feedback data gathered from patients when applying the inventive method, wherein the machine learning model is trained further while being used.

According to a further aspect of the invention, an alert is sent to the medical professional in case a low compliance is predicted.

A rather low value of the predicted compliance for executing the number of exercises the next time can be used as an early warning signal that the patient's condition is deteriorating. In particular, with regard to stroke patients, a possible deterioration of the patient's condition could be an alarm signal indicating further stroke events. When the prediction of a low compliance triggers an alert to be sent to the medical professional, the medical professional gets this information rather early, preferably at a time at which an examination of the patient would not yet reveal any symptoms of a deterioration. Thus, there is more time for an intervention to prevent a (possibly severe) deterioration of the patient's condition.

The alert could be automatically sent by the server to the medical professional's client device, preferably using push notification.

Further above, it was stated that a low predicted compliance can be considered to be a likelihood below 50 % that a patient will complete more than 50 % of the number of exercises. With respect to the automatic sending of an alert, the percentage could even be lowered further. For example, an alert could be sent in case the predicted likelihood for a patient to complete more than 50 % of the number of exercises is below 40% or even below 30%.

According to a further aspect of the invention, the therapy program is directed to improving physical and/or mental capabilities of stroke patients.

In other words, the therapy program comprises physical and/or mental exercises to be performed by the patient. This is of particular relevance for stroke patients as exercising on a regular basis can significantly improve a patient's quality of life in that lost skills are re-learned again. It is noted that the therapy program could also be used for improving physical and/or mental capabilities of other patients affected by other neurogenerative diseases such as Parkinson's disease or patients being handicapped due to other reasons such as old age.

According to an aspect of the invention, a machine learning (ML) model is based on a gradient boosting algorithm and has been adapted to be used in an inventive method for remotely monitoring a patient.

The inventors have found that for remotely monitoring a patient, in particular for predicting the future compliance of a patient to the patient's exercise program, a machine learning model that is implemented by a gradient boosting algorithm, in particular an extended gradient boosting algorithm, is most suitable.

According to a further aspect of the invention, the model is an extended gradient boosting algorithm and has been adapted

by setting the learning rate to a value, which is equal to or larger than 0.15, preferably 0.18, and/or equal to or smaller than 0.34, preferably 0.25, more preferably 0,21 and/or
by setting the maximum tree depth to 4 and/or
by setting the number of trees in the ensemble to a value, which is equal to or larger than 200, preferably 280, more preferably 290 and/or equal to or smaller than 350, preferably 320, more preferably 310 and/or
by setting a training dataset size to a value that is equal to or larger than 4000, preferably 4500.

The learning rate, the maximum tree depth, the number of trees in the ensemble and the training dataset size can be considered to be hyperparameters of the model. The learning rate controls the step size at each iteration when the model's parameters are updated during training.

According to an aspect of the invention, a computer program comprises an inventive machine learning model and furthermore, when being executed on a computer, provides an online portal to a medical professional enabling him to assign an exercise program to the patient, wherein the exercise program consists of a number of exercises to be completed by the patient each time in regular time intervals and prompts the patient to send feedback data via a communication network. In particular, the computer program can be adapted to be executed on a server in an inventive system for remotely monitoring a patient.

According to an aspect of the invention, a method for therapeutic treatment of a patient having suffered a stroke or a patient suffering from Parkinson's disease or an elderly patient comprises a step which consists of an application of an inventive method for remotely monitoring a patient.

According to an aspect of the invention, a method for improving a level of patient compliance, comprises a step which consists of an application of an inventive method for remotely monitoring a patient.

According to an aspect of the invention, a method for improving a level of patient compliance, comprises a step which consists of an application of an inventive method for remotely monitoring a patient.

According to a further aspect of the invention, in the method for improving a level of patient compliance, the level of compliance of a patient having suffered a stroke or a patient suffering from Parkinson's disease or an elderly patient is improved.

According to an aspect of the invention, an inventive method for remotely monitoring a patient is used in therapeutic treatment of a patient having suffered from a stroke.

According to a further aspect of the invention, an inventive method for remotely monitoring a patient is used in improving a level of patient compliance, in particular in order to improve the level of compliance of a patient having suffered a stroke or a patient suffering from Parkinson's disease or an elderly patient.

It shall be remarked that an inventive method for therapeutic treatment of patients suffering from neurological diseases such as Parkinson's Disease or having cardiac problems comprises the inventive method for remotely monitoring a patient. Also, an inventive method of treating elderly, frail patients, to which exercises to be performed in regular time intervals are prescribed, comprises the inventive method for remotely monitoring a patient.

According to an aspect of the invention, a system for remotely monitoring a patient comprises:

a server containing a database,
at least one therapist client to be used by a therapist and
at least one patient client to be used by a patient and configured to present an exercise program to the patient, wherein the exercise program consists of a number of exercises to be completed by the patient each time in regular time intervals,
wherein the patient client is configured to send feedback data to the server via a communication network,
wherein the server is configured to send requests to the patient client via the communication network, the requests prompting the patient to send feedback data to the server, preferably each time after having finished an exercise,
wherein a machine learning (ML) model based on a gradient boosting algorithm is stored on or connected to the server,
wherein the machine learning (ML) model is configured to analyse the feedback data of a patient and to predict the patient's compliance when performing the exercise program the next time after expiry of a number of time intervals to follow, preferably after expiry of the next time interval.

According to a further aspect of the invention, the system is configured to implement an inventive method for remotely monitoring a patient.

According to a still further aspect of the invention, an online portal (Web portal) is provided by the server enabling a medical professional to assign an exercise program to the patient.

According to a still further aspect of the invention, the communication between each two of the database, the therapist client and the patient client is possible in both directions using preferably the WebSocket protocol.

According to an aspect of the invention, a method of predicting physiological parameter values of patients, in particular of patients receiving remote medical attendance, comprises:

providing a measurement device adapted to determine, preferably automatically, at least one value of at least one physiological parameter, when the device has been fitted on or in the patient's body,
prompting the determination of at least one actual value of at least one physiological parameter by means of the measurement device and
transferring the determined at least one actual value to a machine learning model, preferably via a communication network,
wherein by means of the machine learning model a future value of the at least one physiological parameter at a time after the expiry of a specified time interval after the determination of the at least one actual value is predicted,
wherein the machine learning model is a gradient boosting algorithm, in particular a Light gradient boosting algorithm.

Here, prompting can mean instructing the patient in advance when to initiate the determination of a physiological parameter. For example, the patient could be instructed at which times on a day the patient should usually initiate a determination, e.g. by starting the measurement device and/or by taking a reading provided by the measurement device. On the other hand, the term "prompting" is also meant to cover an automatic initiation of a determination, e.g. by automatically starting the measurement device and/or automatically taking a reading provided by the measurement device.

According to a further aspect of the inventionthe physiological parameter is blood pressure such as the systolic and diastolic value thereof, a heart rate, a blood sugar level, blood alcohol content, a level of any other drug in the patient's blood, a blood oxygen level, a lactate level, etc.

According to a further aspect of the invention, the specified time interval is one or more days. However, the specified time interval could also be one, two, three, four, five or six hours.

According to a still further aspect of the invention, the communication network is the internet.

Further features and practicalities of the invention will arise from the description of embodiments based on the attached drawings.

Fig. 1    illustrates two different train-test split methods applicable for assessing a machine learning model's performance.

Fig. 2    shows the ROC (Receiver Operating Characteristic) curves obtained for different models in order to illustrate the performance of the learning models when applied to predict the future compliance.

Fig. 3    shows the dependence of the accuracy of an extended gradient boosting model on the value of the learning rate as model hyperparameter.

Fig. 4    shows the dependence of the accuracy of an extended gradient boosting model on the value of the maximum depth of individual decision trees as model hyperparameter.

Fig. 5    shows the dependence of the accuracy of an extended gradient boosting model on the number of decision trees in the ensemble as model hyperparameter.

Fig. 6    shows a learning curve in order to provide a visual representation of the extended gradient boosting model's performance with respect to the amount of training data.

Fig. 7    schematically shows a hardware setup for implementing the inventive method.

In the following, the inventive approach will be described on the example of a remote treatment of stroke patients.

A therapy program for a stroke patient usually consists of courses to be performed by the patient e.g. over one year. Here, a course includes a set of exercises to be performed by the patient on a regular basis, usually once a day for e.g. 28 days.

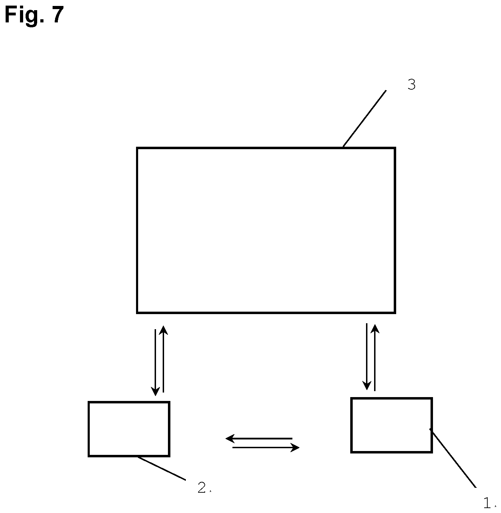

Fig. 7 shows a schematic example of an inventive system for implementing the online therapy. The setup includes a server 3 containing a database and providing an online portal 1, to which a therapist can log on by means of a therapist client, and a patient client 2 to be used by a patient. Communication between each of the server, the therapist client and the patient-side client 2 is possible in both directions using preferably the WebSocket protocol, which is illustrated by double arrows. The WebSocket protocol is advantageous in that real-time communication between the apps installed on the clients and the server is possible. Moreover, latency is considerably reduced compared to a pure Hypertext Transfer Protocol (HTTP). Finally, WebSockets are efficient in terms of message size and header information and WebSockets enable bidirectional data transfer, allowing the server to push updates to the apps, and facilitate seamless back-and-forth communication.

Of course, there may be several therapist clients and several patient clients concurrently connected to the server.

The database stores the individual therapy programs of the patients. Moreover, the database contains feedback information given by the individual patients and containing details on the exercises they performed each day. Finally, the database has access to a machine learning model either implemented on a dedicated computing device or implemented on the server.

The therapist client 1 gives a therapist access to the database, e.g. using a Web dashboard. In particular, it allows the therapist to assemble an individual therapy program for a patient, wherein the therapy program is compiled using predefined exercises stored in the database in order to define individual courses or using predefined courses stored in the database. It is also possible to allocate a predefined therapy program already stored in the database to the patient. Moreover, the therapist client 1 allows the therapist to access feedback data given by the patient and to receive information provided to the therapist by the machine learning model. Finally, the therapist may communicate directly with the patient e.g. by sending messages to the patient or by opening a chat.

The patient-side client 2, e.g. a smartphone or tablet, has a dedicated app installed on it, which provides a framework to present a therapy program with all its elements. The patient can only start working with the app via an invitation that is automatically sent once a therapist onboards a patient in the dashboard and allocates a therapy program to the patient.

The app guides the patient via a chat through the exercises and anything that needs to be done during the program. In addition to that, patients may receive personalized chat messages from their therapists, and are able to contact them via the same chat. Ideally, patients cannot change anything in the app. The app starts every day with the program for that day. If a patient skips a day, the program just continues the next day. After each exercise, patients are prompted to give feedback on the patient's performance of the exercise.

In order to improve the quality of life of stroke patients, it is vital to encourage them to routinely perform their exercises and to do short-term adaptions of the exercises or of the course depending on individual needs of the patient. According to the present approach, the feedback data given by the patient are analysed using a machine learning model, which then provides the therapist with a prediction to which extent the patient will comply with the therapy program in the future, in particular the next day, the day after the next day, etc.

The inventors have tested several differing machine learning models with regard to their ability to reliably predict the future compliance of the patients. the machine learning models tested included the following models:

- Logistic Regression
- Naive Bayes Classifiers
- Support Vector Machines (SVM)
- Random Forest
- XGBoost
- LightGBM
- other decision tree-based models than the foregoing

In the tests, the models were fine-tuned aiming for an increase of the prediction accuracy, leading to the unforeseeable result that a gradient boosting algorithm, in particular an extreme gradient boosting algorithm, will provide the highest percentage of correct predictions of the future compliance. In the following, for illustration purposes a study based on only three different machine learning models is described in more detail:

For the study, anonymized data were collected from 187 patients using a patient-side client (the above-described app) across six medical centers situated in Germany, the Netherlands, and the Philippines. The therapy program consisted of 36 different courses, each with a duration ranging from 28 to 42 days, and patients were required to complete exercises on a daily basis. The dataset contained 7399 instances and the variables that were used are defined in the subsequent table 1.

Table 1

| Variable Name | Variable Description |
| --- | --- |
| Patient ID | ID of the patient |
| Course ID | The course allocated to the patient |
| Center | The medical center competent for the patient |
| Course day | The day number since the starting of the course |

(continued)

| Variable Name | Variable Description |
| --- | --- |
| Target body part | The body part targeted by exercises on that day |
| mRS group | The level of disability group of the patient |
| Day | Day of week |
| Compliance | Did the patient complete more than half of exercises on that day |

With regard to the level of disability group to which a patient belongs, the modified Rankin Scale (mRS) serves as a widely adopted measure for evaluating the extent of disability among stroke patients. Comprising six distinct levels, this scale spans from 0 (reflecting normal motor function) to 5 (indicating severe disability). For the study, each patient was allocated to a specific mRS group and received a tailored therapy program. For the study, the variable 'Compliance' contained two values, namely '0' and '1', with the value '1' indicating that a patient had successfully completed over 50% of the prescribed exercises and the value '0' indicating that the patient did not.

In order to obtain the dataset for assessing the performance of the machine learning models, a series of data preprocessing steps, including variable extraction and aggregation, was applied to the raw dataset. It is worth noting that the dataset did not contain any missing values. In the original dataset, compliance values labeled as "1" accounted for 52% of the total, while compliance values labeled as "0" constituted the remaining 48%. This balance in the values of the output variable helps prevent class imbalance issues that could potentially skew a model's predictions.

The individual variables in the dataset underwent a transformation known as one-hot encoding. One-hot encoding is a method used to represent variables in a numerical format suitable for machine learning algorithms. This technique involves creating binary columns representing the values a variable can obtain. Each binary column corresponds to a specific variable, and for each observation, only one of these columns is "presence" (set to 1) to indicate the presence of that value, while the others are "absence" (set to 0). This is a fundamental step in preparing the variables to be used in the machine learning task.

In order to test the machine learning models with regard to their ability to reliably predict the future compliance of the patients, each model was fed with records of a training dataset. As the goal was to reliable predict the expected patient compliance one, two or three days ahead, the dataset itself comprised the desired output values in that it contained the temporal evolution of the value of the Compliance variable for each patient.

This illustrative study assesses three different machine learning models with regard to their ability to correctly predict the future compliance of a patient: a Random Forest (RF) algorithm, an Extended Gradient Boosting (XGBoost) algorithm and a Light Gradient Boosting Machine (LightGBM or LGBM) using the libraries scikit-learn, XGBoost and LightGBM. All three models are ensemble learning models. Ensemble learning models are accurate and robust by combining diverse models such that the collective performance is superior to the performance of any single model. All three models that were tested use decision tree learning. Whereas in the Random Forest algorithm right from the beginning an ensemble of decision trees is created, in the two gradient boosting models decision trees are added sequentially, wherein each new tree is trained to correct the errors made by the previous tree. The LightGBM model differs from the XGBoost model in that trees are not grown level-wise but leaf-wise. Whereas in XGBoost all nodes in one level are split, in LightGBM only the leaf that will provide the maximum reduction in the loss function is split.

In order to adjust (fine-tune) the three machine learning models to the task to be achieved, which is predicting the future compliance, three different hyperparameters were adjusted:

- Number of Trees:
  This hyperparameter determines the number of base models (trees) in the ensemble. A higher number of trees can capture more complex patterns in the data but may lead to overfitting if set too high.

- Max Depth of Trees:
  The maximum depth of individual decision trees in the models.

- Learning Rate:
  This hyperparameter is specific to gradient boosting algorithms like XGBoost and LightGBM. It controls the step size at each iteration while updating the model's parameters.

Evaluating the performance of the learning models is a crucial step in assessing their effectiveness. Several evaluation metrics were selected to measure the quality of the models' predictions.

Accuracy is the most straightforward metric, measuring the proportion of correctly predicted compliances (true positives) out of the total number of predicted compliances in the dataset. Precision and Recall are often used together to assess the models ability to correctly predict compliances (true positives) while minimizing false positives or negatives. Precision measures the proportion of true positive predictions among all positive predictions, while Recall calculates the proportion of true positives among all actual positives.

A further metric was the F1-score which is the harmonic mean of Precision and Recall. It is especially useful when there is a trade-off between Precision and Recall, as it takes both into account. A higher F1-score indicates a better balance between Precision and Recall.

$$Accuracy = \frac{\text{Number of Correct Predictions}}{\text{Total Number of Predictions}} \quad (1)$$

$$Recall = \frac{\text{True Positives}}{\text{True Positives+False Negatives}} \quad (2)$$

$$Precision = \frac{\text{True Positives}}{\text{True Positives+False Positives}} \quad (3)$$

$$F1 - Score = 2 \cdot \frac{\text{Precision·Recall}}{\text{Precision+Recall}} \quad (4)$$

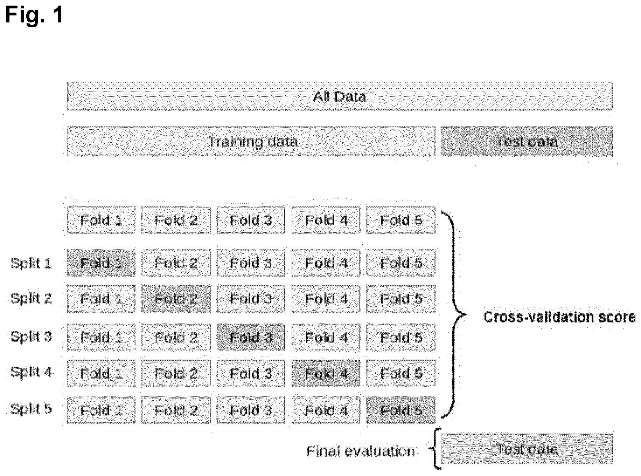

Two different strategies were employed to assess the models concerning data partitioning. As illustrated in the upper part of Fig. 1, the first approach involved a train-test split method. In this setup, 80% of the data constituted the training set, utilized to train the model. Subsequently, the remaining 20% comprised the test set, employed to evaluate the model's performance using the previously mentioned metrics. The second strategy adopted a 5-fold cross-validation technique. As illustrated in the lower part of Fig. 1, in this approach the training set is divided into five subsets (or folds), with each fold serving as a test set once while the remaining four folds are used for model training. This process is repeated five times, ensuring that each fold serves as the test set exactly once. The accuracy of the model was calculated for each iteration, and the average accuracy across all iterations was computed, referred to as the cross-validation score. This approach provided a robust estimate of the model's performance by considering various data partitions and reducing the risk of evaluation bias.

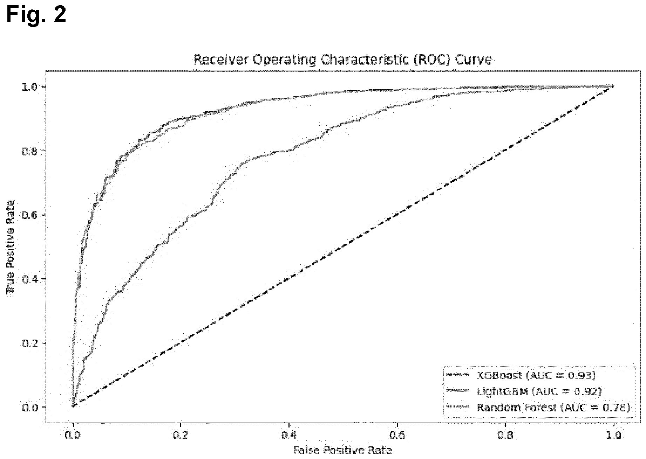

Fig. 2 illustrates the performance of the learning models when applied to predict the future compliance by showing the ROC (Receiver Operating Characteristic) curves obtained for the models. The area under the ROC curve (AUC) is a widely used metric to assess the power of classification models. As can be seen (see also Table 3), the Random Forest model achieved an AUC of 0.78, an accuracy of 0.69 and an F1-Score of 0.74, whereas the LightGBM model exhibited an AUC of 0.92, an accuracy of 0.84 and an F1-Score of 0.85 and the XGBoost model excelled with an AUC of 0.93, an accuracy of 0.86 and an F1-Score of 0.86.

Table 3

| Model | Metrics | | | |
|---|---|---|---|---|
| | Accuracy Score | F1 Score | Precision Score | Recall Score |
| Random Forest | 0.69 | 0.74 | 0.64 | 0.87 |
| XGBoost | 0.86 | 0.86 | 0.84 | 0.88 |
| LightGBM | 0.84 | 0.85 | 0.83 | 0.87 |

From Fig. 2 and Table 3 it results that gradient boosting algorithms are the learning models of choice for predicting the future compliance of patients performing their prescribed daily exercises. Moreover, it turned out that an extended gradient boosting algorithm gives the best results.

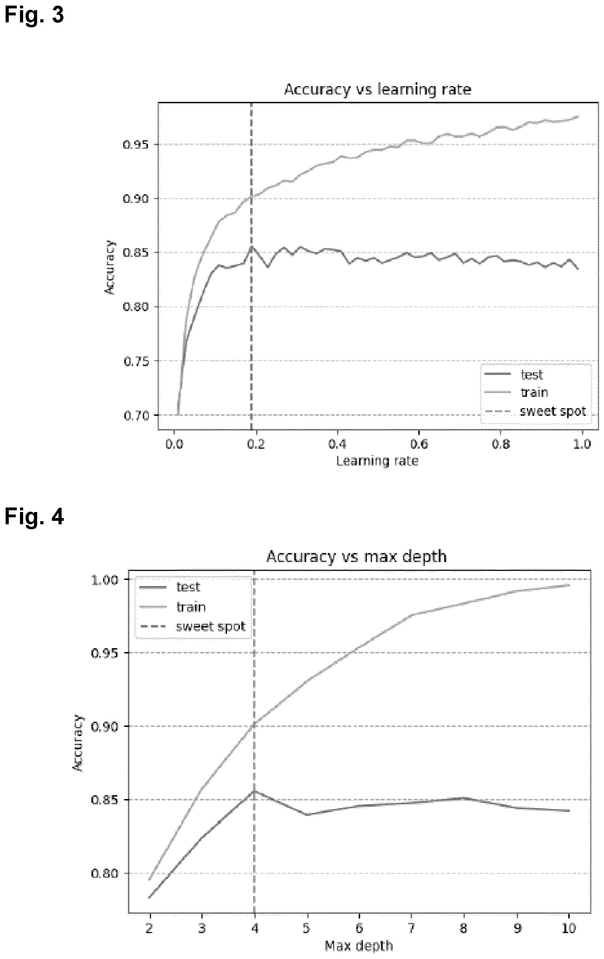

In order to find the optimum hyperparameters for an extended gradient boosting algorithm, we varied the three hyperparameters mentioned further above. Each of Figs. 3 to 5 shows the dependence of accuracy from a corresponding hyperparameter value. From the figures it is apparent that a learning rate equal to or larger than 0.15, a depth of trees equal to or larger than 4 and a number of trees equal to or larger than 250 should be chosen when applying the extended gradient boosting algorithm. In order to strike a balance between minimizing training accuracy and maximizing test accuracy to effectively prevent overfitting, values for the hyperparameters that are close to the lower limits of the above-mentioned ranges are preferable. The inventors consider a learning rate between 0,18 and 0.21, a maximum tree depth of 4 and a number of trees in the ensemble that is equal to or larger than 290 and/or equal to or lower than 310 as optimum values for the hyperparameters. In Figs. 3 to 5, the values 0,19, 4 and 290 are designated as "'sweet spots".

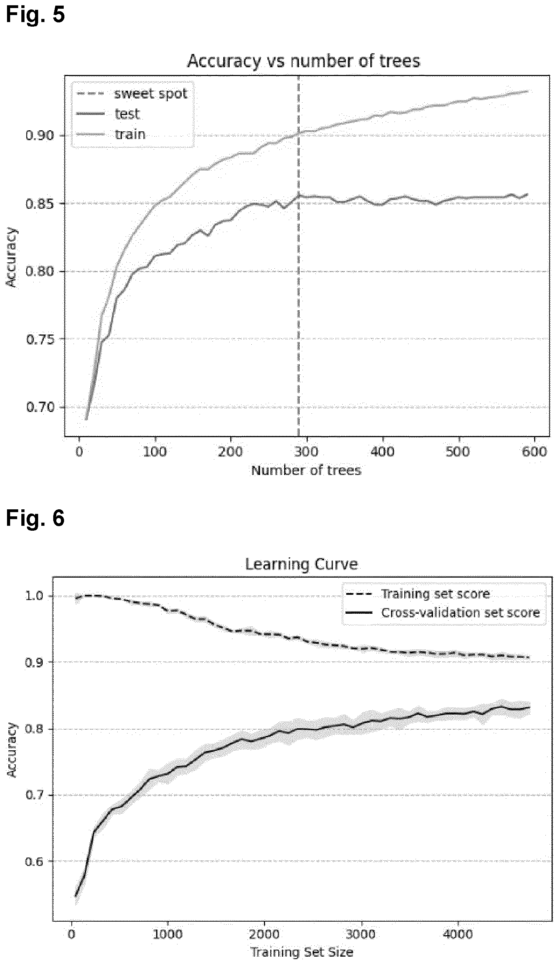

The learning curve of Fig. 6 provides a visual representation of the extended gradient boosting model's performance with respect to the amount of training data. At the initial stages of training, the cross-validation score exhibited a lower value, while the training accuracy was exceptionally high, suggesting that the model was essentially memorizing the training data, a classic sign of overfitting. However, as the training dataset size increased, a notable trend emerged. The cross-validation score gradually improved indicating the model's enhanced capacity to generalize effectively to new and diverse data samples, while the training accuracy began to decrease. This decrease reflects the model's transition from initially memorizing training data to learning more meaningful patterns.

It turned out that the favourite machine learning model to be used for predicting patients' future compliance is a gradient boosting algorithm, in particular an extended gradient boosting algorithm, e.g. using XGBoost. When such a model has been trained, e.g. using a training dataset described further above, the model is able to provide a therapist with a (for example binary) value reflecting a predicted compliance or non-compliance of a patient when practicing the prescribed exercises on the next day and/or the day after the next day and/or one of the following days. In particular, the model analyses the compliance history of a patient by accessing the patient's datasets of a number of days, wherein each dataset looks like the one shown in Table 1. The predicted compliance for a patient is then added to the patient-specific data in the database.

As a result, a therapist can easily access the predicted values via the dashboard presented in the Web portal and can proactively intervene in order to keep a patient at practising his/her exercises inspite of a possibly negative outlook given by the machine learning model. For example, the therapist could either encourage the patient by opening a chat or triggering an automated encouragement message. Here, Push notification can be used for contacting the patient, ensuring that the app on the patient client will inform the patient, even when the app is not running or when the app is in an idle background state. Alternatively or in addition, the therapist could adapt the exercises, e.g. by changing to easier exercises or by changing to an easier course.

Accordingly, due to the use of the inventive machine learning model, the overall efficacy of the stroke recovery intervention can be improved in that adherence to the therapy program is improved.

The prediction accuracy for future compliance of the patients can be improved by including physiological parameters (such as blood pressure data) in the feedback data and considering the same when predicting the future compliance. Apart from that, based on values of physiological parameters in the feedback data, in accordance with the invention a machine learning model could predict future values (e. g. one day, two days, three days ahead, etc.) of a patient's physiological parameter(s). Such predicted future values are valuable information for a therapist, allowing a therapist to identify a deterioration of a patient's condition at an early stage. In the following, an example of an inventive prediction of future values of a patient's physiological parameters is described on the example of blood pressure values.

In order to predict future values of a patient's physiological parameters, the setup shown in Fig. 7 that was described further above can be used, wherein of course it is also possible to gather a patient's feedback data and communicate with the patient by means of that setup.

As was the case in the example above, which was related to the prediction of a patient's future compliance, several differing machine learning models were assessed with regard to their ability to reliably predict future values for a patient's blood pressure. This was done in a study using a dataset consisting of data collected from 73 patients.

The patients were equipped with monitoring devices to track their blood pressure (both systolic and diastolic) in addition to heart rate, wherein the patients were instructed to take three readings at three different times a day, e.g. in the morning, at noon and in the evening. In some cases, specific messages prompting the client. The recorded values were stored on the patient-side client 2 and the mean as well as the standard deviation of the three readings of a day were computed. The number of instances in the dataset was 5600, wherein the dataset's variables are detailed in table 2. It should be mentioned here that it is also conceivable that the monitoring devices are connected to the communication network so that the server itself can initiate a measurement and record the readings.

Table 2

| Variable Name | Variable Description |
|---|---|
| Patient ID | ID of the patient |
| Date | The date on which the readings were taken |
| Avg systolic | The average systolic BP on that day |
| Systolic std | The standard deviation of the three systolic BP records |
| Avg diastolic | The average Diastolic BP on that day |

(continued)

| Variable Name | Variable Description |
|---|---|
| Diastolic std | The standard deviation of the three Diastolic BP records |
| Avg heart rate | The average heart rate on that day |
| Heart rate std | The standard deviation of the three heart rate records |

Across the variables of the dataset, the ratio of missing values ranged from approximately 6% to 11%. Accordingly, an imputation method was used for replacing missing data with substituted values.

As the imputation method will have an influence on the predictive power, in particular prediction accuracy, the following imputation methods for time series data were assessed:

- Mean imputation and median imputation
- Linear interpolation
- Last observation carried forward (LOCF) which replaces each missing value with the most recent present value prior to it.
- Moving average and moving median which is the average / median over certain time window.
- a K-Nearest Neighbour algorithm.

To assess the different imputation methods, a subset of the dataset comprising 2,891 instances without any missing values was selected. To simulate real-world scenarios, 15% of null values were introduced randomly for each variable in the selected subset. The null values were imputed using the different imputation techniques, and the MAPE (mean absolute percentage error) was calculated between the imputed values and the actual values. Here, LOCF performed least effectively, exhibiting the highest level of inaccuracy. Conversely, linear interpolation, KNN (with k = 5), moving average, and moving median with a time window of 5 days demonstrated relatively similar and improved imputation performance. However, the inventors found that the best methods were the mean and the median imputation methods, wherein missing values were replaced with the mean value and median value, respectively, in all other cases, with a slight advantage observed for the median approach. Thus, the missing values were imputed with the median before training the models.

Evaluating the performance of different learning models for predicting future values for a patient's blood pressure, the following evaluation metrics were applied:

The Mean Absolute Error (MAE) corresponds to the average absolute difference between a model's predictions and the actual target values (desired output values). MAE provides a straightforward measure of the model's accuracy in terms of the magnitude of errors, wherein a lower MAE indicates better predictive accuracy.

$$MAE = \frac{1}{n}\sum_{i=1}^{n} |y_i - \widehat{y_i}|$$

The Mean Absolute Percentage Error (MAPE) is a variation of MAE expressed as a percentage of the actual value. It is particularly useful for understanding the magnitude of the error relative to the actual values. Like MAE, a lower MAPE indicates a better predictive accuracy.

$$MAPE = \frac{1}{n}\sum_{i=1}^{n} |\frac{y_i - \widehat{y_i}}{y_i}| \times 100$$

The Coefficient of Determination (R-squared or R2) is a metric that quantifies the pro-portion of the variance in the target variable that is explained by the model. R2 ranges from 0 to 1, where a higher R2 indicates that the model captures a larger portion of the variance.

$$R2 = 1 - \frac{\sum_{i=1}^{n}(y_i - \widehat{y_i})^2}{\sum_{i=1}^{n}(y_i - \overline{y_i})^2}$$

Concerning data partitioning for training and testing the models, the test set comprised the most recent three readings, whereas the training set encompassed all the remaining data.

When predicting physiological parameters, the problem to be solved is different from the one described above that referred to a binary value for the future compliance in that there are usually no discrete values that the variable can attain but there exists a continuous range of values for the variable to be predicted. Nevertheless, the inventors examined the suitability of the same machine learning models assessed above for predicting a patient's future compliance.

Surprisingly, the inventors found that also for predicting the patients' blood pressure values, gradient boosting algorithms provided the best results. Using MAE (MAPE %) for evaluation, Table 4 shows the performance of an extended gradient boosting algorithm (XGBoost) for predicting the blood pressure values and the heart rate (designated as "target variables") one, two and three days ahead.

Table 4

| Target Variable | Days Ahead | | |
|---|---|---|---|
| | One day | Two days | Three days |
| Systolic BP | 6.3 (5.04% | 6.38 (5.11%) | 6.3 (5.12%) |
| Diastolic BP | 4.37 (6.04% | 4.28 (5.92%) | 4.3 (6.04%) |
| Heart Rate | 4.23 (6.03% | 4.27 (6.12%) | 4.3 (6.24%) |

Table 5 shows the corresponding results for the use of a Light Gradient Boosting Machine (LightGBM).

Table 5

| Target Variable | Days Ahead | | |
|---|---|---|---|
| | One day | Two days | Three days |
| Systolic BP | 6.31 (5.07%) | 6.36 (5.11%) | 6.3 (5.12%) |
| Diastolic BP | 4.35 (6.03%) | 4.28 (5.92%) | 4.3 (5.99%) |
| Heart Rate | 4.26 (6.08%) | 4.26 (6.12%) | 4.3 (6.24%) |

Finally, Table 6 shows the average R2 Score for both gradient boosting models.

Table 6

| Model | Days Ahead | | |
|---|---|---|---|
| | One day | Two days | Three days |
| XGBoost | 0.659 | 0.658 | 0.653 |
| LightGBM | 0.659 | 0.662 | 0.654 |

It can be seen that both models exhibit similar predictive capabilities, with a slight advantage for LGBM. However, an additional significant advantage of LGBM is its considerably faster computation time. Consequently, a Light gradient boosting algorithm is the preferred machine learning model for predicting physiological parameters such as blood pressure and heart rate.

**Claims**

1.   A method for remotely monitoring a patient, comprising:

providing an online portal to a medical professional enabling him to

- assign an exercise program to the patient, wherein the exercise program consists of a number of exercises to be completed by the patient each time in regular time intervals and
- prompting the patient to send feedback data via a communication network,

wherein patient's compliance to the exercise program is predicted by a machine learning (ML) model based on a gradient boosting algorithm,

wherein the machine learning model analyses the feedback data of a patient and predicts the compliance when performing the exercise program the next time after expiry of a number of time intervals to follow.

2. The method of claim 1, wherein the compliance is predicted by determining the likelihood of a patient completing more than 50%, preferably more than 70%, of the number of exercises when performing the exercise program the next time after expiry of a number of time intervals to follow, preferably after expiry of the next time interval.

3. The method of claim 1 or 2, wherein a motivation message is sent to a patient for the case that a low compliance is predicted.

4. The method of one of the preceding claims, wherein the feedback data include one or more of the following data:

   - a patient identifier and optionally a course identifier,
   - on which day of the sequential number of days of the course the feedback data are sent,
   - the date,
   - patient's subjective evaluation whether the patient complied on that date or not,
   - the number of exercises assigned to the patient on that date,
   - the painfulness of at least one exercise,
   - the difficulty the patient had when carrying out at least one exercise,
   - the time needed for at least one of the individual exercises making up the training program,
   - information for at least one of the exercises whether it has been completed or not,
   - the number of exercises completed per day,
   - an exercise identifier,
   - information whether an exercise targets an upper or lower body part,
   - the day of the week the feedback data are sent,
   - the therapy center to which the patient belongs to,
   - the actual number of exercises executed by the patient each time,
   - which of the number of exercises are skipped,
   - the average time per exercise,
   - the patient's assessment of the intensity of one or more exercises,
   - patient's demographics like age, gender, height and weight.

5. The method of one of the preceding claims, wherein the machine learning model has been trained using one or more of the following data:

   - time needed for at least one of the individual exercises making up the training program,
   - information on whether an exercise has been completed or not,
   - the number of exercises completed per day,
   - an exercise identifier,
   - information whether an exercise targets an upper or lower body part,
   - the course that the patient is assigned to,
   - on which day of the sequential number of days of the course the feedback data, are sent
   - the day of the week the exercises were executed,
   - the therapy center to which the patient belongs to,
   - a patient identifier or a course identifier,
   - the length of the time interval,
   - the actual number of exercises executed by the patient each time,
   - which of the number of exercises are skipped,
   - the time needed for at least one of the individual exercises making up the training program,
   - patient's demographics like age, gender, height and weight,

6. The method of one of the preceding claims, wherein the ML model has been trained to determine a level of patient compliance by means of exemplary feedback data.

7. The method of one of the preceding claims, wherein an alert is sent to the medical professional in case a low compliance is predicted.

8. The method of one of the preceding claims, wherein the therapy program is directed to improving physical and/or mental capabilities of stroke patients.

9. A machine learning (ML) model, which is based on a gradient boosting algorithm and has been adapted to be used in a method according to one of claims 1 to 8.

10. The machine learning (ML) model according to claim 9, wherein the model is an extended gradient boosting algorithm and has been adapted

by setting the learning rate to a value, which is equal to or larger than 0.15, preferably 0.18, and/or equal to or smaller than 0.34, preferably 0.25, more preferably 0,21 and/or
by setting the maximum tree depth to 4 and/or
by setting the number of trees in the ensemble to a value, which is equal to or larger than 200, preferably 280, more preferably 290 and/or equal to or smaller than 350, preferably 320, more preferably 310 and/or
by setting a training dataset size to a value that is equal to or larger than 4000, preferably 4500.

11. A method for therapeutic treatment of a patient having suffered a stroke or a patient suffering from Parkinson's disease or an elderly patient comprising a step of applying a method according to one of claims 1 to 8.

12. A method for improving a level of patient compliance, comprising a step of applying a method according to one of claims 1 to 8.

13. The method of claim 12, wherein the level of compliance of a patient having suffered a stroke or a patient suffering from Parkinson's disease or an elderly patient is improved.

14. Use of a method as defined in any one of claims 1 to 8 in therapeutic treatment of a patient having suffered from a stroke.

15. Use of a method as defined in any one of claims 1 to 8 in improving a level of patient compliance, in particular in order to improve the level of compliance of a patient having suffered a stroke or a patient suffering from Parkinson's disease or an elderly patient.

# Fig. 1

## Fig. 2

Receiver Operating Characteristic (ROC) Curve

**Fig. 3**

Accuracy vs learning rate

**Fig. 4**

Accuracy vs max depth

## Fig. 5

Accuracy vs number of trees

## Fig. 6

Learning Curve

**Fig. 7**

## EUROPEAN SEARCH REPORT

**Application Number**

EP 23 22 0563

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/108086 A1 (UNIV CALIFORNIA [US]) 15 June 2023 (2023-06-15) * figure 2A * * paragraph [0024] * * paragraph [0030] - paragraph [0031] * * paragraph [0078] * * paragraph [0083] * * paragraph [0093] * * paragraph [0100] - paragraph [0103] * * paragraph [0147] - paragraph [0151] * ----- | 1-15 | INV. G16H20/30 |
| X | US 2022/148730 A1 (NAUMOV MICHAEL [IL] ET AL) 12 May 2022 (2022-05-12) * paragraph [0004] * * paragraph [0135] * * paragraph [0198] * ----- | 1-15 | |

TECHNICAL FIELDS SEARCHED (IPC)

G16H

The present search report has been drawn up for all claims

| Place of search | Date of completion of the search | Examiner |
|---|---|---|
| Munich | 24 April 2024 | Gardiner, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 22 0563

This annex lists the patent family members relating to the patent documents cited in  the above-mentioned European search report.
The members are as contained in the European Patent Office EDP file on
The European Patent Office is in no way liable for these particulars which are merely given for the purpose of information.

24-04-2024

| Patent document cited in search report | Publication date | Patent family member(s) | | Publication date |
|---|---|---|---|---|
| WO 2023108086 A1 | 15-06-2023 | NONE | | |
| US 2022148730 A1 | 12-05-2022 | AU | 2021363603 A1 | 15-06-2023 |
| | | CA | 3199311 A1 | 28-04-2022 |
| | | CN | 117083681 A | 17-11-2023 |
| | | EP | 4233064 A1 | 30-08-2023 |
| | | IL | 302240 A | 01-06-2023 |
| | | JP | 2023546221 A | 01-11-2023 |
| | | KR | 20230091963 A | 23-06-2023 |
| | | US | 2022148730 A1 | 12-05-2022 |
| | | WO | 2022084408 A1 | 28-04-2022 |

EPO FORM P0459

For more details about this annex : see Official Journal of the European Patent Office, No. 12/82